# EUROPEAN PATENT APPLICATION

(11) **EP 4 768 909 A1**
(43) Date of publication of application: **01.07.2026**
(21) Application number: 25221269.1
(22) Date of filing: 05.12.2025
(51) Int. Cl.: G01N 33/18, C02F 1/00

(54) **SYSTEMS AND METHODS FOR PROVIDING DYNAMIC WATER QUALITY DETERMINATION OF A WATER SYSTEM**

(30) Priority: 31.12.2024 US 202419006580
(71) Applicant: Mettler-Toledo Thornton, Inc., Billerica MA 01821 (US)
(72) Inventor: Mukhida, Samir, Glendale, AZ-85308 (US); Bevilacqua, Anthony, Medford, MA 02155 (US); McLean, Robert, Manchester, NH 03109 (US); Yadav, Mayank, Philadelphia, PA 19130 (US); Reijnders, Anjan, Billerica, MA 01821 (US)
(74) Representative: Mettler-Toledo

(57) **Abstract**

Systems and methods for dynamically determining water quality for a water system are disclosed. A controller includes, and/or has access to, electronically stored thresholds separating zones representing states of the water system, one of the thresholds ("composite threshold") is specific to a composite water quality metric ("CWQM") and a plurality of the thresholds ("characteristic thresholds") are each specific to a respective characteristic of a sample of the water system. The controller receives data from sensors for measuring the characteristics, and determines, for each of the characteristics, a proximity of an associated measurement(s) to an associated characteristic threshold. The controller uses the determined proximities to adjust the CWQM, and determine a current state of the water system based thereon relative to the composite threshold, a representation of which is communicated to electronic device(s).

## Description

### TECHNICAL FIELD

Exemplary embodiments relate generally to systems and methods for providing dynamic water quality determinations for a water system, in particular a water purification system.

### BACKGROUND AND SUMMARY OF THE INVENTION

Clean, high-quality water is critical for a variety of applications, from drinking water to pharmaceuticals, manufacturing (e.g., semiconductor), and power production (e.g., for steam generation), by way of non-limiting example. At least some of these applications are sufficiently critical to warrant enhanced regulation, control, and/or certification. For example, in the U.S. pharmaceutical industry, water purity standards are generally defined, at least in part, by the United States Pharmacopeia (USP) and enforced by the United States Food and Drug Administration (FDA). Similar national regulatory bodies control the water purity standards and enforcement for drug manufacturing abroad. In addition to governmental regulatory bodies, water quality standards are also frequently defined by industry groups, and most manufacturers of purified water maintain proprietary internal water quality standards to optimize the process control for their manufacturing equipment and usage loops. To ensure regulatory compliance, industrial compliance, and internal quality compliance, water system operators employ a variety of analytical techniques to control the process of generating and maintaining purified water.

For example, the USP has defined limits for what constitutes sufficiently pure water for safe use in life sciences production facilities. These limits include maximum concentrations of Total Organic Carbon (TOC), viable organisms and endotoxins, maximum electrical conductivity (representative of ionic impurities), and requirements to protect against added substances like sodium bisulfite and ozone. Other pharmacopoeias have similar requirements, while some have other acceptance criteria.

To ensure compliance with regulatory bodies, life science manufacturers are generally required to meet specific acceptance criteria on sensors, analyzers, and all measuring devices used for these high purity water applications to assure their performance such as accuracy, lifetime, repeatability, or the like. These measurements of specific chemical, microbial and physical attributes may also have specific limits. These limits generally range from voluntary (e.g., from industry) to recommended to required (e.g., USP or Nuclear Regulatory Commission).

Controls for the microelectronics water segment are generally not regulatory-driven - rather, they are promulgated by industry trade groups, and are specified by individual users to meet their specific needs. In the microelectronics segment, by way of non-limiting example, ultrapure water is typically used to rinse semiconductor wafers or used to prepare chemical solutions. Any chemical (organic, ionic, metallic, etc.), micro-organism, or particle left on the wafer may be detrimental to the performance of the wafer, such as by risking a short circuit as a result of the contamination. Measurement of these chemicals/particles is sometimes accomplished by data collection using various sensors and analyzers. Unfortunately, these data are often only used as lagging indicators of water quality, and serve more of a reactive process control role, in some cases after the damage to a production batch is done.

Key analytics for water purification, distribution, and water quality vary per application. Common water treatment processes and systems comprise three generic functions: pre-treatment, purification, and storage and distribution (sometimes with additional purification). There are several challenges with the traditional approach to process analytics of water purification, storage, and distribution, including but not necessarily limited to, those further described herein.

Process analytics data interpretation and subsequent translation into corrective actions requires considerable skill. Effective water system operators must have deep knowledge of their water systems and every purification step therein, be skilled at analysis of large data sets, have process and compliance expertise, and possess deep technical Root Cause Analysis (RCA) skills. This combination of skills can be hard to find and is often distributed across several functions. Moreover, this reliance on subject matter experts results in water quality variability across sites and industries as there is no standardized comprehensive metric for overall purified water quality.

Studying previous raw measurement data offers a historical view of water quality, resulting in a reactive approach to process control instead of a proactive approach. Even if limit alarms are triggered nearly instantaneously, they are still a lagging indicator of water quality, often requiring immediate action. If the root cause of an out-of-limit water parameter cannot be immediately addressed, purified water manufacturers may find themselves out of industrial, regulatory (e.g., governmental), or internal compliance. This can result in costly production delays, product loss due to low yield, and occasionally requires costly product recalls.

Not all water analytics measurements produce online, real-time, and continuous data, resulting in discontinuous data streams that further complicate a rapid response to water quality anomalies. Some techniques are inherently slow, such as microbial detection via growth promotion-based plate counts, which takes multiple days to produce results, and can have high costs associated with frequent testing.

These disclosures reduce the complexity of water system diagnostics and offer solutions to all three challenges, among other advantages. By providing an overall water quality metric that takes account of many or all water purification, storage, and distribution analytics, the health and compliance state of a water treatment system can be understood at once. Moreover, by providing predictive indicators for water quality and compliance state, optionally including recommended actions to be taken by the user and/or automated process controller to correct and/or control water quality and/or compliance state, process control shifts from a reactive discipline to an actions-based predictive and proactive discipline. This allows users to take preventative actions on root causes identified, improve system uptime, and minimize events resulting in out-of-compliance operation, among other advantages. By, optionally, allowing for the inclusion of both online and offline data, the water quality indicator and action logs are driven by up-to-date data, offering real-time, continuous, and actionable feedback to the end user.

In exemplary embodiments, without limitation, data from sensors of or for the water system are gathered, such as on a real-time, or at least substantially real-time (e.g., accounting for normal transmission and processing times, such as without an intentional delay of more than 10 minutes) basis, ongoing basis (e.g., continuously, every X seconds, minutes, etc.). The data from each of the sensors is analyzed to determine measurements for characteristics (e.g., chemical, physical, and/or microbiological) of the water. Each of the measurements are compared against an associated, respective one of characteristic-specific thresholds (characteristic threshold) as the thresholds are typically different for the various characteristics (e.g., the acceptable level of TOC may be different from the acceptable level of bioburden and/or the two may be measured in different units). Each of the thresholds separates zones such that each one of the one or more measurements for a respective one of the characteristics falls into one of at least two zones. Each of the zones may correspond to a state of the respective characteristic. In this way, a state of each of the characteristics may be determined from the measurements. The zones are preferably arranged in a progression of criticality. Multiple thresholds may optionally be provided for each characteristic, such as to define three or more zones, and a comparison of the measurement(s) for the respective characteristics may be made relative to the multiple associated thresholds.

A proximity of a current set of one or more measurements to the associated critical characteristic threshold (or a (next) most critical characteristic threshold) is determined for each of the characteristics. Weightings may be determined based on proximity to the associated critical characteristic threshold (or a (next) most critical characteristic threshold), for example such that less proximate measurements (to the associated critical characteristic threshold or the (next) most critical characteristic threshold) are weighted more heavily and *vice-versa,* and applied. In an embodiment, without limitation, proximity may be determined with regard to a next most critical threshold in the progression of criticality towards the critical or most critical threshold. As further described herein, measurements may be transformed into "scores" prior to or after comparison against the thresholds. The proximity analysis is further described herein. Weightings are applied to the measurements or scores, and the resulting weighted measurements or scores are numerically combined, such as by summing, to arrive at a composite water quality metric (CWQM). The CWQM is compared against a composite-specific threshold (composite threshold) or thresholds to determine a current state of the water system (e.g., water purification and/or distribution system). In this way, the overall health of the water system may be assessed, such as to determine which zone the system is in.

Data from the sensors, which may provide the measurements, may be processed or further processed, such as into "scores", before, while, and/or after applying the weightings to the measurements, and/or before and/or while, and/or after the combining the scores. These processed measurements may sometimes be referred to herein as "scores" and/or "measurements". Such processing (of the data and/or measurements) may include various data interpretation, manipulation and/or standardization techniques, such as but not necessarily limited to, unit conversions, scaling, interpretations, transformations, derivations, combinations thereof, or the like. Such processing may, optionally, be specific to the zone in which the measurement falls.

In exemplary embodiments, without limitation, for each of the characteristics, the associated one or ones of the characteristic thresholds are transformed to associated one or ones of threshold scores of a preset range of scores, and/or the associated one or ones of the measurements are transformed to associated one or ones of measurement scores of the preset range of scores, wherein the proximity of the associated one or ones of the measurements to the associated one or ones of the characteristic thresholds is based on a difference between the associated one or ones of the measurement scores and the associated one or ones of the threshold scores

By way of non-limiting example, the zones may include a compliance, alert, action, and breach zone, where delineation between the zones is established by three thresholds (one defining the boundary between compliance and alert, another between alert and action, and so forth). As another example, without limitation, an in compliance - no action, in compliance - take action, and out of compliance zones may be established by two thresholds. The number of thresholds and associated zones is preferably the same across the characteristics and/or relative to the CWQM, though they may differ. In particular, the number of thresholds and zones may be the same across the characteristics and the CWQM, wherein the zones associated with each of the characteristics and the CWQM indicate the same states of the water system with respect to the respective characteristic and the CWQM, respectively. The thresholds and/or zones may be defined, at least in part, based on various regulatory body requirements, which may be geographic specific. The thresholds and/or zones may be adjusted by users and/or geography of the system.

Where a single threshold is utilized, it is considered the critical threshold. Where multiple thresholds are utilized, the threshold separating a most critical of the zones (e.g., out of compliance) is considered the most critical threshold, and the threshold that is encountered next on the progression of criticality towards the critical or most critical threshold is the next most critical threshold.

The weighting may be provided such that, where measurement(s) for one or more of a first subset of the characteristics (e.g., those which are regulated by relevant authority; sometimes referred to herein as "required measurements" or "required parameters") falls on an adverse side of the associated critical characteristic threshold (or next, as the case may be) most critical characteristic threshold), the resulting CWQM necessarily falls on the adverse side of the composite threshold (or (next) most critical composite threshold). This may be achieved, in one exemplary embodiment without limitation, by weighting a least proximate one of the measurement(s) associated with the first subset of the characteristics that fall(s) on the adverse side of the associated threshold (i.e., a most unfavorable measurement), with 100% of the weight, by way of non-limiting example. In this regard, the proximity of measurement(s) falling on the favorable side of the associated threshold may, optionally, be ignored or disregarded for purposes of determining the CWQM.

Further, the weighting may be provided such that, where all of the measurement(s) for the first subset of the characteristics fall on a favorable side of the associated critical characteristic threshold (or (next) most critical characteristic threshold), the resulting CWQM necessarily falls on the favorable side of the composite threshold (or (next) most critical composite threshold). This may be achieved by weighting each of the measurement(s) based on their respective proximity to the critical characteristic threshold (or (next) most critical characteristic threshold), by way of non-limiting example. Further still, the weighting may be provided such that, even where one or more, up to all, of measurement(s) for a second subset of the characteristics (e.g., those which are unregulated; sometimes referred to herein as "supplemental measurements" or "supplemental parameters") fall on the adverse side of the critical characteristic threshold (or (next) most critical characteristic threshold), the resulting CWQM necessarily still falls on the favorable side of the critical composite threshold (or (next) most critical composite threshold) so long as all of the measurement(s) for the first subset of characteristics fall on the favorable side of the respective critical characteristic threshold (or (next) most critical characteristic threshold). Stated another way, only the measurement(s) for the characteristics in the first subset may drive the CWQM to the adverse side of the critical composite threshold (or (next) most critical composite threshold). The measurement(s) for the characteristics in the second subset may still influence the CWQM, such as within the non-most-critical zone(s). This may be realized by, for example and without limitation, establishing and applying a minimum value to the measurements or scores for the second subset of the characteristics.

The adverse side of the threshold may be the side (e.g., above or below or inside/outside) associated with the critical or the (next, as the case may be) most critical zone in the progression of criticality (e.g., an out-of-compliance zone or closer to the out-of-compliance zone). The favorable side of the threshold may be the side (e.g., above or below or inside/outside) associated with a least or next less critical zone in the progression of criticality (e.g., the in-compliance zone(s) or further from the out-of-compliance zone). While thresholds are sometimes discussed, other bounding parameters, which are not necessarily thresholds, may be utilized.

Optionally, the nomenclature for the zones and/or thresholds of the first and second subset of characteristics may be different such that those of the second subset do not have an out-of-compliance zone or associated threshold, even if associated with multiple zones and/or thresholds (e.g., an alert and action zone and associated threshold, but no out-of-compliance zone or threshold).

The analysis may be undertaken on a real-time, or at least substantially real-time basis, ongoing basis (e.g., continuously, every X seconds, minutes, etc.). Data may be utilized from a variety of sensors associated with a variety of water system components, such as part of an active use system. In this way, the health of the system and/or various characteristics thereof may be determined dynamically.

A listing of certain embodiments is further provided herein and incorporated by reference.

Further features and advantages of the systems and methods disclosed herein, as well as the structure and operation of various aspects of the present disclosure, are described in detail below with reference to the accompanying figures.

### BRIEF DESCRIPTION OF THE DRAWINGS

In addition to the features mentioned above, other aspects of the present invention will be readily apparent from the following descriptions of the drawings and exemplary embodiments, wherein like reference numerals across the several views refer to identical, similar, or equivalent features, and wherein:
**FIGURE 1** is a schematic for an exemplary water system;
**FIGURE 2** is a schematic of exemplary components of the figure 1 water system;
**FIGURE** 3 is a schematic for an exemplary water system with an exemplary dynamic water quality monitoring system, such as for use with the water system and components of figures 1-2;
**FIGURE 4** is a plan, schematic view of an exemplary system for providing dynamic water quality determinations, such as for the water system of figure 1-2;
**FIGURE 5** is a flow chart with exemplary logic for operating the system of figure 4 or comprised in the water system of figure 3;
**FIGURE 6** is a flow chart with further exemplary logic for the method of figure 5 and/or operating the system of figures 3 and/or 4;
**FIGURE 7** is a flow chart with further exemplary logic for the method of figures 5-6 and/or operating the system of figures 3 and/or 4;
**FIGURE 8A** is a plan view of an exemplary graphical output for the systems and methods of figures 3-7;
**FIGURE 8B** is a plan view of another exemplary graphical output;
**FIGURE 8C** is a plan view of another exemplary graphical output;
**FIGURE 8D** is a plan view of another exemplary graphical output; and
**FIGURE 8E** is a plan view of another exemplary graphical output.

### DETAILED DESCRIPTION OF EXEMPLARY EMBODIMENT(S)

Various embodiments of the present invention will now be described in detail with reference to the accompanying drawings. In the following description, specific details such as detailed configuration and components are merely provided to assist the overall understanding of these embodiments of the present invention. Therefore, it should be apparent to those skilled in the art that various changes and modifications of the embodiments described herein can be made without departing from the scope and spirit of the present invention. In addition, descriptions of well-known functions and constructions are omitted for clarity and conciseness.

Embodiments of the invention are described herein with reference to illustrations of idealized embodiments (and intermediate structures) of the invention. As such, variations from the shapes of the illustrations as a result, for example, of manufacturing techniques and/or tolerances, are to be expected. Thus, embodiments of the invention should not be construed as limited to the particular shapes of regions illustrated herein but are to include deviations in shapes that result, for example, from manufacturing.

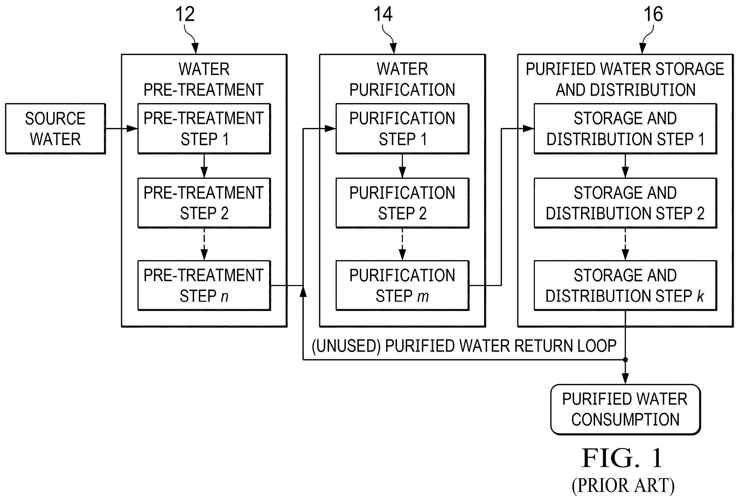

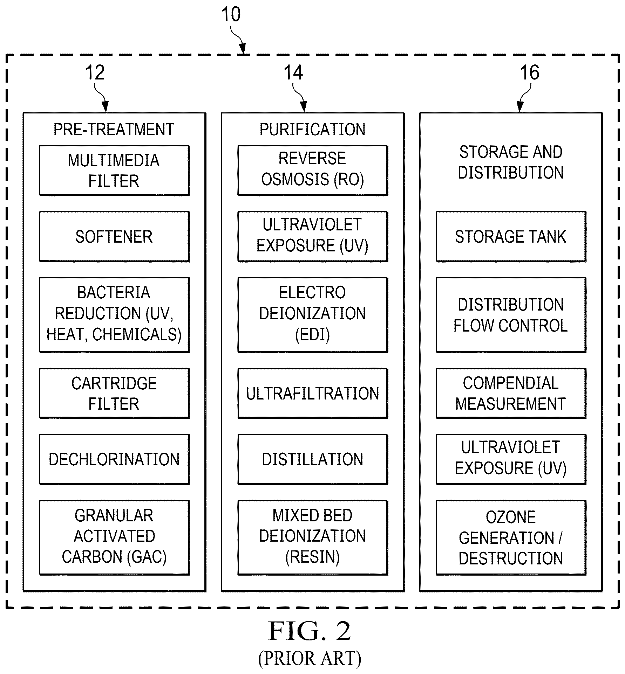

**FIGURE 1** and **FIGURE 2** illustrate an exemplary, prior art water system 10, including various exemplary pretreatment 12, purification 14, and storage and distribution 16 subsystems and/or steps thereof.

Key components and/or steps for a conventional water system 10 vary per application. However, most water systems 10 comprise three generalized phases and/or subsystems: pre-treatment 12, purification 14, and storage and distribution 16, sometimes with added purification steps. Unused water may be returned to storage and distribution or further upstream in the purification system. Each phase/subsystem includes a number of water processing (e.g., purification) steps and/or components. For example, typical pre-treatment 12 steps and/or components include filtering with a multimedia filter, water softening with a water softener, and adsorption of impurities with granular activated carbon (GAC), by way of non-limiting example. Typical purification steps and/or components include reverse osmosis, single- or mixed-bed deionization, and viable microbial reduction (e.g., via UV exposure), by way of non-limiting example. Typical storage and distribution steps and/or components include additional purification steps, compendial measurements, distribution and flow control, and further microbial reduction, by way of non-limiting example. Depending on quality, unused purified water is typically returned to the pretreatment 12, water purification 14, and/or storage and distribution 16 for reuse. Waste water may be returned to pretreatment 12 and/or water purification 14 for re-purification and re-use. A single water treatment solution might feed multiple distribution loops and/or end applications.

To determine the efficiency and the efficacy of each processing step, offline and/or online analytics are sometimes employed. For example, to test the efficacy of bacterial reduction (via UV exposure, ozonation, or heat treatment, for example), the offline technique of plate counting by growth promotion of viable organisms may be used (measuring the quantity of microbial colonies formed from viable organisms in a specific volume of water), or the online technique of Light Induced Fluorescence detection may be used, such as in real time (measuring autofluorescence of organisms, such as in auto fluorescent units - AFU per unit volume). Other analytics to test treatment efficacy include electrical conductivity (a measure of ions or ionic impurities), TOC, particle counting, water flow and temperature, and individual ionic impurity detection via analyzers measuring the concentration of silica, sodium, chloride, sulfate, oxygen, ozone, or the like, in water. The foregoing examples are not intended to be limiting. These measurements, and optionally many more chemical, physical, and microbial measurements, constitute the parameters that determine the quality of the water. The quality of the water impacts criteria such as safety, product performance, and process efficiency.

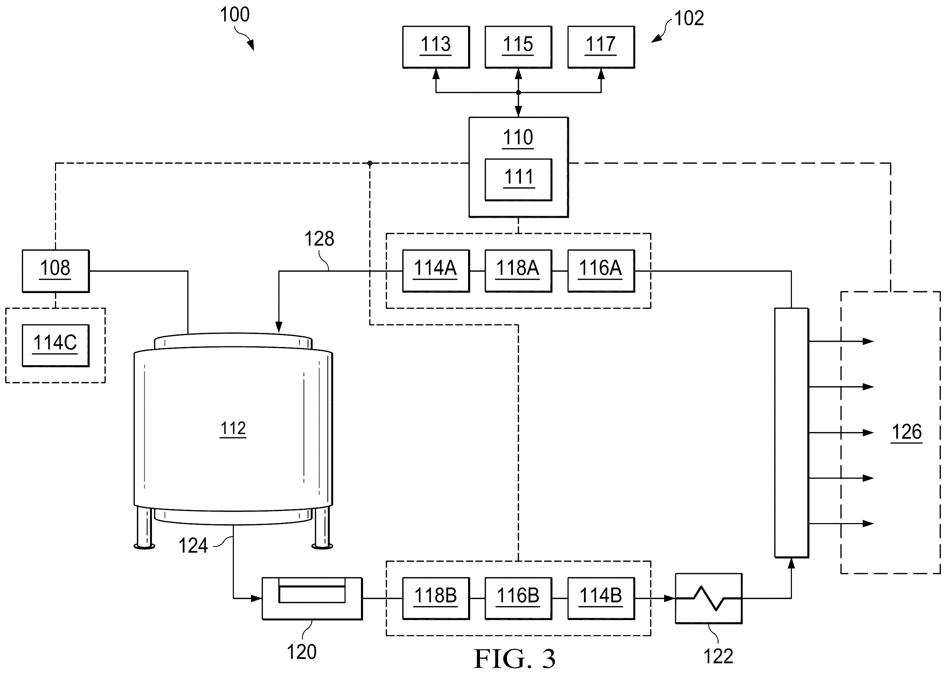

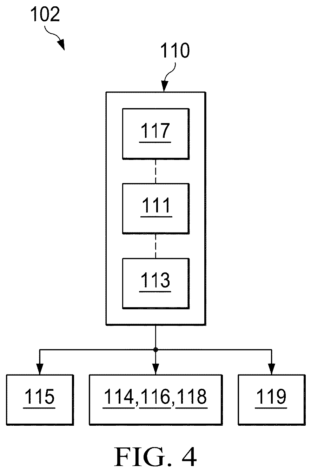

**FIGURE 3** illustrates an exemplary water system 100 (e.g., the water system 10 as shown and/or described with regard to figure 1-2, or a different type and/or kind of water system) comprising an exemplary system 102 for providing dynamic water quality determinations (herein also "system"), shown more particularly in **FIGURE 4****.** The system 102 comprises an analysis subsystem 110 according to any embodiment disclosed further below. According to the exemplary embodiment of the system 102 shown in figure 4, the analysis subsystem 110 comprises a controller 111 and a communication device 113. The communication device is configured to enable communication between the analysis subsystem 110, in particular the controller 111, sensors 114, 116, 118 and, optionally, various components of the water system 100 as described in more detail further below and an electronic device 119 that causes representation of an outcome of the dynamic water quality determination, in particular of a current state of the water system. The exemplary embodiment of the system 102 shown in figure 4 comprises further one or more non-transitory electronic storage devices 115, and the communication device 113 is configured to enable communication between the controller 111 and the one or more non-transitory electronic storage devices 115. In the exemplary embodiment shown in figure **4****,** at least one of the non-transitory electronic storage devices 115 is remote from the analysis subsystem 110 for enabling remote control of parameters, such as thresholds, needed for dynamic water quality determination. The exemplary embodiment of the system 102 shown in figure 4 comprises further one or more processors 117 that may be part of the analysis subsystem 110, for example by being the controller 111 or by being part of the controller 111. The non-transitory electronic storage device(s) 115 may comprise software instructions, which when executed by the processor(s) 117 and/or other processors, causes functionality shown and/or described herein to be carried out by such processors and/or related components, algorithms, thresholds, parameters, historical data (e.g., regulated parameter data for subsequent auditing, other sensor 114, 116, 118 data, other water system 10, 100 operations data, combinations thereof, or the like), tensor(s), combinations thereof, or the like. As mentioned, the communication device 113, non-transitory electronic storage device(s) 115, and/or processor(s) 117 may be part of, or remote and in wired and/or wireless connection with, the analysis subsystem 110. The communication device 113 may facilitate, by way of non-limiting example, remote access to data, water system 10, 100 operations, analysis subsystem 110 operations (e.g., required parameters, thresholds, state information, historical data review, combinations thereof, or the like), combinations thereof, or the like. Such communication may be direct or by way of one or more intermediary components (e.g., PLCs).

The system 102 may be applied to an existing water system, provided as part of a new water system, and/or as part of an upgrade to an existing water system, by way of example. In exemplary embodiments, without limitation, purified water may be provided from a water purification subsystem 108, which may include some or all of the components and/or utilize some or all of the steps of known water systems 10 and/or methods, such as those shown and/or described in figures 1-2 by way of non-limiting example. The purified water may be stored at one or more storage tanks 112. Various sensors and/or analyzer devices (sometimes referred to herein generally as "sensors" or "analyzers") may be provided to measure various characteristics of the water at various stages of the water system 100. For example, without limitation, a bioburden analyzer 114 may be provided which measures bioburden of water from the water purification subsystem 108 supplied to and/or located at the storage tank 112. All or some of the various sensors and/or analyzers devices may be part of the system 102, in particular if the water system does not comprise the sensors and/or analyzers needed for generating the data indicating measurements of the characteristics on which the water quality determination bases.

The water stored at the storage tank 112 may be provided by way of a pressurized feed line 124 to a point of use (POU) subsystem 126, such as for use. Sensor(s), such as but not limited to, a bioburden analyzer 114, a conductivity analyzer 118, and/or a total organic carbon (TOC) analyzer 116 may be provided along, or otherwise in fluid connection with, the feed line 124. Optionally, one or more water treatment devices, such as but not limited to, an ultraviolet light source 120 and/or heat exchanger 122, may be provided. These devices may alter characteristics of the water during use. A return line 128, such as for unused water, may be provided from the POU subsystem 126 and/or end application to the storage tank 112. One or more sensors, such as but not limited to, the bioburden analyzer 114, the conductivity analyzer 118, and/or the TOC analyzer 116 may be provided along, or otherwise in fluid connection with, the return line 128.

The number, type, and/or arrangement of components of the water system 100, including those for storage, distribution, and/or treatment as well as sensing water quality, are exemplary and are not intended to be limiting. For example, without limitation, multiple water purification subsystems 108, storage tanks 112, feed lines 124, return lines 128, POU subsystems 126 and/or end applications may be utilized. As a further example, without limitation, the water system 100 may comprise one or more pipes, lines, valves, pumps, any or all of the components shown and/or described with respect to figures 1-2, combinations thereof, or the like, by way of non-limiting example. Alternatively, or additionally, various types, kinds, and/or arrangements of the sensor(s) 114, 116, 118 may utilized. For example, without limitation, the sensor(s) 114, 116, 118 may include the conductivity analyzer 118, an ozone sensor, the TOC analyzer 116, the bioburden analyzer 114, a pressure sensor, a flow rate sensor, a temperature sensor, a pH sensor, combinations thereof, or the like by way of non-limiting example.

The sensor(s) 114, 116, 118 may be in electronic connection with the analysis subsystem 110. In exemplary embodiments, without limitation, the sensor(s) 114, 116, 118 include those associated with a user specified and/or certified portion of the water system 100 for demonstrating regulatory compliance.

The analysis subsystem 110 may, optionally, be in electronic connection with various components of the water system 100, such as pumps, valves, sanitization devices and/or subsystems, thermal regulation devices and/or subsystems, physical security (e.g., door locks), combinations thereof, or the like which control operations of the water system 100 and/or water purification subsystem 108 thereof. In this way, the analysis subsystem 110 may, optionally, be configured to operationally control flow of water within at least part of the water system 100 and/or operations of the water purification subsystem 108, providing the ability to halt production and/or use, by way of non-limiting example. For example, without limitation, the analysis subsystem 110 may be configured to operate one or more of the pumps, valves, sanitization devices and/or subsystems, thermal regulation devices and/or subsystems, physical security (e.g., door locks), combinations thereof, or the like to halt production, isolate water, isolate access, combinations thereof, or the like, such as where the current state of the water system is determined to be on the adverse side of the threshold for the CWQM.

The system 102, in particular the analysis subsystem 110, may be configured for controlling the water quality of a water system, for example a water system as disclosed with respect to figures 1-3, such as in at least one of a more efficient and a more reliable manner. For example, without limitation, the analysis subsystem 110 may be configured to change, or instruct changes to, an operation parameter or a setting of the water system, such as but not necessarily limited to, at least one of: a state of a pump, valve, sanitization device and/or subsystem, thermal regulation device and/or subsystem, physical security (e.g., door locks, combinations thereof, or the like).

Commonly, the sensor(s) 114A, 116A, 118A associated with required parameters are those provided along return line 128 as those readings are typically required to show compliance. Data from remaining sensor(s) 114B, 116B, 118B, and/or 114C if any, are associated with supplemental parameters. However, this is merely exemplary and not intended to be limiting. In exemplary embodiments, the analysis subsystem 110 would be programed to associate readings with certain of the sensor(s) 114, 116, 118 as supplemental or required, such as on a user specified basis in accordance with the user's compliance certification scheme.

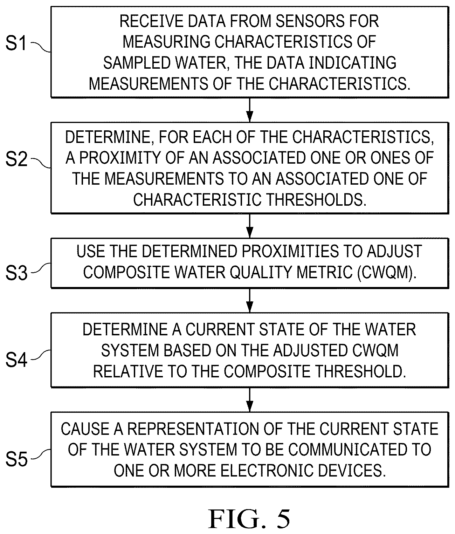

**FIGURE 5** through **FIGURE 7** illustrate exemplary logic for operating the system 102 for providing dynamic water quality determinations for a water system, and particularly (or exclusively) the analysis subsystem 110 thereof. Data may be received at the analysis subsystem 110 from the various sensor(s) 114, 116, 118. The data may be stored at one or more databases of, or in electronic connection with, the analysis subsystem 110.

The sensor(s) 114, 116, 118 may gather data points to be used as measurements/parameters for measuring water quality. All parameters can be defined as variables, such as P₁ through Pₙ, where n is the total number of parameters available. The data from the sensor(s) 114, 116, 118 may be processed, as needed, to arrive at measurements for characteristics of the analyzed sample. Such processing may include, for example without limitation, scaling, conversion, filtering, combination, combinations thereof, or the like. Such processing may be performed at the sensor(s) 114, 116, 118 and/or the analysis subsystem 110. Data from a single or multiple of the sensor(s) 114, 116, 118 may be utilized to provide a given measurement for a given characteristic. For example, without limitation, parameters may be a measurement of an individual characteristic (such as pH); a combined measurement such as differential pressure (Pout - Pin) across a component of the water system 100 (e.g., filter); a statistic of the measurement (like running average or standard deviation) over a duration of time; a computed time-based measurement such as a trendline; or a computation of a future value (or state) of the measurement, or a computation of a future time when an adverse event is projected.

The analysis subsystem 110 may be configured to account for delay in time between data points from the various sensor(s) 114, 116, 118 such that a same, or substantially the same, sample of the flow is analyzed.

The measurements may be described as a collection of time dependent orthogonal vectors, where the direction of the vector is associated with a characteristic (e.g., unique water quality or process quality attribute, such as species of contaminant or process attribute like flow rate), and the magnitude of the vector describes the severity of water quality or process quality deterioration. A magnitude of 0 may describe an ideal state and deviations from 0 in the positive or negative direction may scale with the degree of water quality or process control change. Each vector may serve as a proxy for water quality or process quality, while a combination of vectors may provide a composite measure of water quality. Each of these vectors may be time dependent, and every updated parameter measurement may update the associated vector. All vectors combined may span a space that defines composite water quality.

After data collection, all data (e.g., vectors) may be mapped into a tensor that is stored in a database. As further discussed herein, certain measurements may be associated with characteristic thresholds designated as required (herein such thresholds sometimes described as required thresholds, and measurements sometimes described as required measurements), and may be grouped together, while others may be associated with characteristic thresholds designated as supplemental (herein such thresholds sometimes described as supplemental thresholds, and measurements sometimes described as supplemental measurements) and may be separately grouped together. This database may be utilized by the analysis subsystem 110 to obtain information on individual water quality and process quality parameters, both for current state and as a function of time, and to generate a CWQM as further discussed herein.

Every new tensor may be processed by a water quality analysis module (WQAM), which may include an expert system algorithm (ESA). The WQAM may be configured to transform the tensor into a single CWQM based on a fusion of net contributions of individual measurement to water quality in relation to pre-defined thresholds. Operation of the WQAM may be as shown and/or described with particular regard to any of figures 5-7 and/or further set forth herein, in exemplary embodiments, without limitation.

The analysis subsystem 110 and/or one or more associated databases may store the pre-defined thresholds. One of these thresholds may be specific to the CWQM (composite threshold). Some or all of a remainder of these thresholds may each be specific to a respective one of the characteristics (characteristic thresholds).

The required thresholds and/or associated water quality characteristics (hereinafter also sometimes referred to as the "regulated water characteristics") may be those set by a user or authority. The required thresholds and/or regulated water characteristics may reflect regulated and/or critical water quality characteristics and/or requirements for purified water (e.g., bioburden levels, total organic carbon; hereinafter also sometimes referred to as the "regulated water characteristics for purified water"). The required thresholds and/or regulated water characteristics for purified water may be geographically specific. For example, without limitation, the required thresholds and/or regulated water characteristics for purified water may reflect those promulgated by USP and/or enforced by the FDA and/or other global regulators and/or pharmacopoeia. Other standards may be utilized. These required thresholds and/or regulated water characteristics for purified water often, but not necessarily, are associated with one or more of the following characteristics: TOC, conductivity, bioburden, endotoxin, and the concentration of any added substances such as dissolved ozone. The required thresholds and/or regulated water characteristics for purified water may be predetermined, though they may alternatively or additionally be manually set and/or modified.

The supplemental thresholds may reflect unregulated and/or less critical water quality characteristics. The supplemental thresholds may be geographically specific. For example, without limitation, the supplemental thresholds may reflect those promulgated by various trade associations. Other standards may be utilized. The supplemental thresholds may be associated with the same type(s) of characteristics as those associated with the required thresholds, but may be associated with sensor readings from non-critical locations in the water system (e.g., conductivity measured before and after two resin beds in the purification phase, or TOC measurements during purification). The supplemental thresholds may, alternatively or additionally, be associated with one or more of the following characteristics: other chemical properties such as pH, dissolved oxygen, turbidity, etc., and physical properties such as water temperature, particle concentration, flow rate, pressure, tank levels, etc. The supplemental thresholds may be predetermined, though they may alternatively or additionally be manually set and/or modified.

Each of the thresholds may separate zones, which may be arranged in a progression of criticality. For example, each threshold may define two zones (e.g., in compliance, out of compliance) for each characteristic and/or for the CWQM. Alternatively, multiple thresholds may be associated with each characteristic and/or the CWQM to define three or more zones for each characteristic and/or the CWQM (e.g., breach, action, alert, compliance; out of compliance, in compliance - take action; in compliance - no action). In this regard, while a single threshold is sometimes shown and/or described herein, such as to define two zones, multiple thresholds for a given characteristic or CWQM may be utilized.

The number and type of thresholds and zones is preferably the same across all characteristics and the CWQM, though they may differ. Various number and names of the zones may be utilized.

The measurements for each characteristic may be compared against the associated characteristic threshold. The proximity of the measurement(s) for each of the characteristics to the associated threshold may be determined.

In exemplary embodiments, without limitation, proximity is determined, for each of the characteristics (or at least each characteristic associated with a required parameter), with respect to the associated critical or most critical threshold associated with a worst performing characteristic. For example, without limitation, if there are four characteristics (characteristics #1-4), each of the four characteristics being, for example, in one of the "compliance" zone, the "alert" zone, the "action" zone, or the "breach" zone, and characteristic #3 is the worst performing one at a given point of time and is in its "alert" zone, the relevant characteristic threshold for all characteristic may nevertheless be taken as the characteristic threshold that separates the "breach" zone from the "action" zone. In this way, proximity is standardized.

In an alternative exemplary embodiment, without limitation, proximity may be determined with regard to a next most critical threshold associated with a worst performing characteristic, where the next most critical threshold is the characteristic threshold that is encountered next on the progression of criticality towards the critical or most critical threshold. In the aforementioned example, without limitation, this would be the characteristic threshold that separates the "alert" zone from the "action" zone, which is the next threshold encountered on the way to the "breach" zone. Then, for each of the characteristics #1-4, the "proximity" is determined for the associated characteristic threshold that separates the "alert" zone from the "take action" zone. In this way, proximity is simplified and/or more accurately determined.

The supplemental thresholds may be established such that the supplemental measurements may fall on the adverse side of the supplemental thresholds. However, in exemplary embodiments, without limitation, the weightings are established such that any one or more (up to all) of the supplemental measurements falling on an adverse side of the supplemental threshold (e.g., falling closer to out-of-compliance), or a most critical one of the supplemental thresholds where multiple supplemental thresholds are utilized, cannot (at least by itself) cause the CWQM to fall on an adverse side of the composite threshold (e.g., resulting in an out-of-compliance CWQM determination), or most critical composite threshold. Stated another way, only the required measurements may drive the CWQM to the adverse side of the composite threshold (or most critical composite threshold). The supplemental measurements may influence the CWQM, such as within the non-most-critical zone(s). In an exemplary embodiment, without limitation, this may be realized by establishing and applying a minimum value for the supplemental measurements above the critical or most critical threshold and/or transforming the supplemental measurements to scores which have a minimum value above the critical or most critical threshold. This may be performed as before and/or as part of the weighting process and/or before and/or part of the combining process to determine the CWQM. In another exemplary embodiment, without limitation, this may be realized by establishing a scale and translating the supplemental parameter measurements into scores based, at least in part, on the scale, such as part of the combination process to determine the CWQM, such that a zero score is provided to correspond to supplemental measurements falling at or on the adverse side of the critical threshold (or the (next) least critical threshold) for the composite threshold. This may prevent such unfavorable supplemental parameter measurements from impacting the CWQM score. In this way, the supplemental parameter measurements may fall into, for example, the non-compliance zone without causing the CWQM score to likewise fall out of the compliance zone, by way of example. This may, alternatively or additionally, provide for smoother changes in the CWQM as measurements change over time.

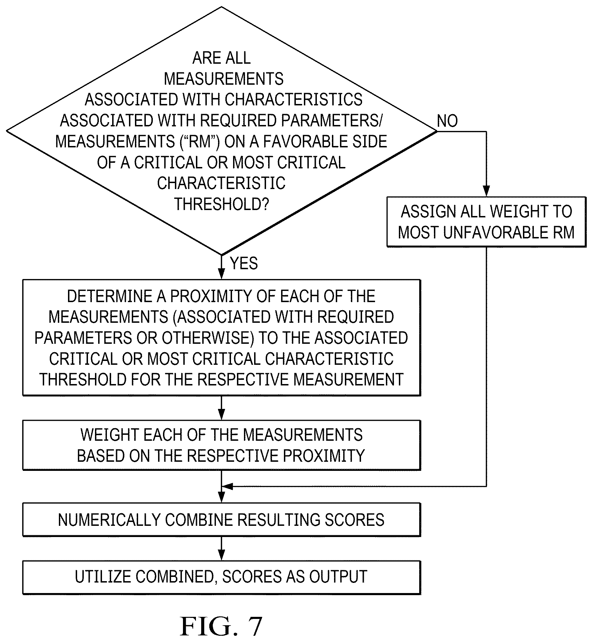

As schematically shown in **FIGURE 7** for a specific, non-limiting case, where one or more, up to all, of the required measurements fall on the adverse side of the associated critical characteristic threshold or (next, as the case may be) most critical characteristic threshold, the one of the required measurements that performs the worst, i.e., the one of the required measurements that falls on the adverse side and is, among the required measurements that fall on the adverse side, most distant from the critical threshold or most critical threshold (sometimes referred to herein as most unfavorable measurement) , may be used by the analysis subsystem 110 as an output, or basis of an output, and/or may be assigned up to 100% of available weighting. This may necessarily result in a CWQM falling on the adverse side of the composite threshold, thereby providing an out-of-compliance CWQM and/or water system state determination. In this way, the single worst performing characteristic may solely drive the CWQM and/or water system state determination, at least until another characteristic performs worse, and/or until all of the required measurements fall on the favorable side of the associated threshold.

Alternatively, this analysis may be made preliminarily such that weighting, consulting supplemental measurements, and/or numerical combination of measurements for at least all characteristic is not required.

Where all of the required measurements fall on a favorable side of the associated critical characteristic threshold (or next, as the case may be) most critical characteristic threshold) (e.g., in compliance), the weightings may be applied based on the respective proximity of each measurement to the associated critical characteristic threshold (or (next) most critical characteristic threshold).

For example, without limitation, up to 100% of available weighting is assigned to the required measurement that performs worst in any case in which a required parameter falls on the adverse side of the associated threshold that separates the most favorable zone, usually the "compliance" zone, from the subsequent less favorable zone. Depending on the number and type of the zones, the subsequent less favorable zone may be, for example, the "alert" zone or the "breach / out-of-compliance" zone. In this way the single worst performing required characteristic may solely drive the CWQM whenever at least one required characteristic is out of the most favorable zone, and the single worst performing required characteristic that solely drives the CWQM may change if another required characteristic starts performing worse. Finally, driving of the CWQM by the single worst performing required characteristic may stop if all of the required characteristics are in the most favorable zone again.

Optionally, the nomenclature for the zones and/or thresholds of the required and supplemental measurements may be different such that supplemental measurements do not have an out-of-compliance zone or associated threshold, even if associated with multiple zones and/or thresholds.

In exemplary embodiments, without limitation, the weightings may be differently scaled depending on whether the respective measurement(s) falls above or below the associated threshold. For example, without limitation, a higher degree of sensitivity may be utilized for measurements on the favorable side of the associated threshold (or next threshold).

The weighted measurements or scores may be numerically combined, such as by summing. The combined, weighted measurements or scores may be used by the analysis subsystem 110 as an output.

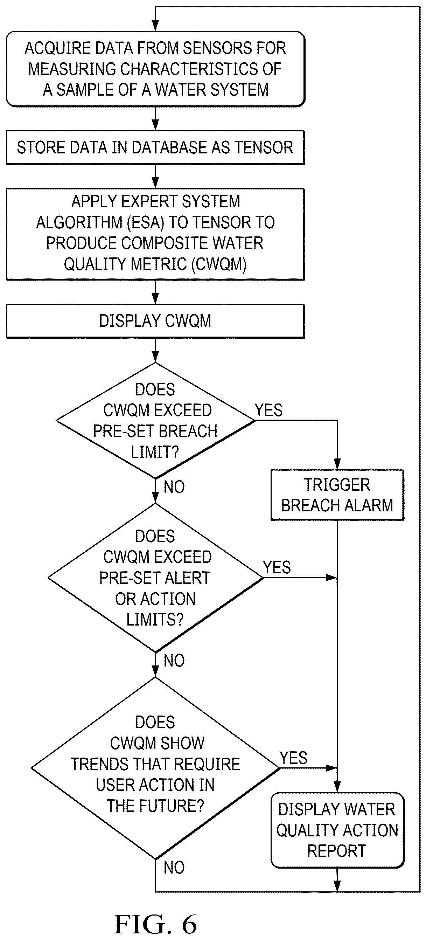

As schematically shown in **FIGURE 6** for a specific, non-limiting case, the output, such as in the form of the CWQM, may be compared against the composite threshold. A state of the water system may be determined based on the location (above or below) and/or proximity of the CWQM to the composite threshold. The output may be communicated to one or more electronic devices. The output may be displayed directly (e.g., as a numeric score) and/or in graphical form, such as a location within one of two or more zones.

A water quality state of the analyzed sample with respect to each characteristic may be determined based on the location (above or below) and/or proximity of the characteristic specific output relative to the characteristic threshold. This water quality state may be used as a stand in for the state of the water being provided, the water system 100, and/or water purification subsystem 108 (e.g., in compliance, out of compliance). The output may be communicated to one or more electronic devices. The output may be displayed directly (e.g., as a numeric score) and/or in various graphical forms, such as a location within one of two or more zones, as further described herein.

Where the CWQM exceeds a pre-set breach limit, for example, a breach alarm may be triggered, such as in the form of one or more electronic notifications transmitted to one or more remote electronic devices, such as by the analysis subsystem 110. A water quality action report may, alternatively or additionally, be displayed and/or communicated. Where the composite water quality metric exceeds other pre-set limit(s) (e.g., alert, action), for example, the water quality action report may be displayed and/or communicated. Alternatively, or additionally, where data trends suggest that user action will be required, such as within a predetermined future time period (e.g., days, weeks, etc.), the water quality action report may be displayed and/or communicated. Data trends may be determined using extrapolation, regression analysis, integral analysis, derivative analysis, combinations thereof, or the like. Trend analysis may be performed on a measurement and/or characteristic specific basis, and/or for the CWQM.

The water quality action report may indicate action(s) that should be taken. The actions may be determined by the WQAM. For example, without limitation, where data from specific sensor(s) 114, 116, 118 results in an exceeded threshold, the analysis subsystem 110 may determine that an associated action is needed (e.g., change particular filter, resin bed, flush system, service pump, combinations thereof, or the like). The associated actions may be predetermined based on stored expert knowledge and retrieved by a query/return analysis. In exemplary embodiments, without limitation, such actions may be carried out on an automated basis, such as under operative control of the analysis subsystem 110.

By way of non-limiting example, an index score of 0 to 100 may be utilized for the CWQM. For each WQAM transformation, the CWQM may be updated, resulting in a time dependent CWQM function. For easier interpretation of the CWQM, its full scale may be divided into pre-set zones. For example, the scale could be divided into zones that indicate the composite water quality is in a state of: compliance, alert, action, and breach. As a more specific example, without limitation, the breach zone may span scores from 0-5, the action zone may span scores from 5-20, the alert zone may span scores from 20-40, and the compliance zone may span scores from 40-100. Other divisions may be utilized, both with additional or fewer zones. The zones may align with those recommended by pharmacopeias around the world for individual parameters.

Net contributions to the CWQM for each characteristic may be determined for each WQAM transformation based on each individual characteristic's proximity to the associated characteristic threshold(s). For required parameters, these thresholds may follow local regulatory standards, or internal quality standards. As individual required parameters get closer to their threshold(s), their weight in the WQAM transformation may dynamically increase. This may ensure that the CWQM is driven by the worst performing required parameter. Like the required parameters, the weight of supplemental parameters may be also dynamically controlled by their proximity to predefined thresholds. The contribution of supplemental parameters to the CWQM may be limited to specific zones. For example, the supplemental parameters may only affect the CWQM score in the compliance zone, whereas the other zones may only be accessed via the performance of required parameters. The thresholds may be predefined and/or user updated.

In other exemplary embodiments, without limitation, the WQAM may be configured to assign all weight to a worst performing required parameter and/or utilize only the worst performing required parameter, such as without a need for weighting and/or consulting the other parameters. This may ensure that the CWQM is entirely driven by the single worst parameter.

The data collection and/or analysis may be made on an ongoing basis (e.g., every X seconds, minutes, etc.), such as in real time or substantially real time. In this way, the quality of the water may be determined dynamically over time. The sensor(s) 114, 116, 118 may be configured to take measurements of an active flow of the water system 100. For example, without limitation, the sensor(s) 114, 116, 118 may be placed along a sample line or portion of a main flow line for the water system 100 and take measurements on an ongoing basis while the flow continues.

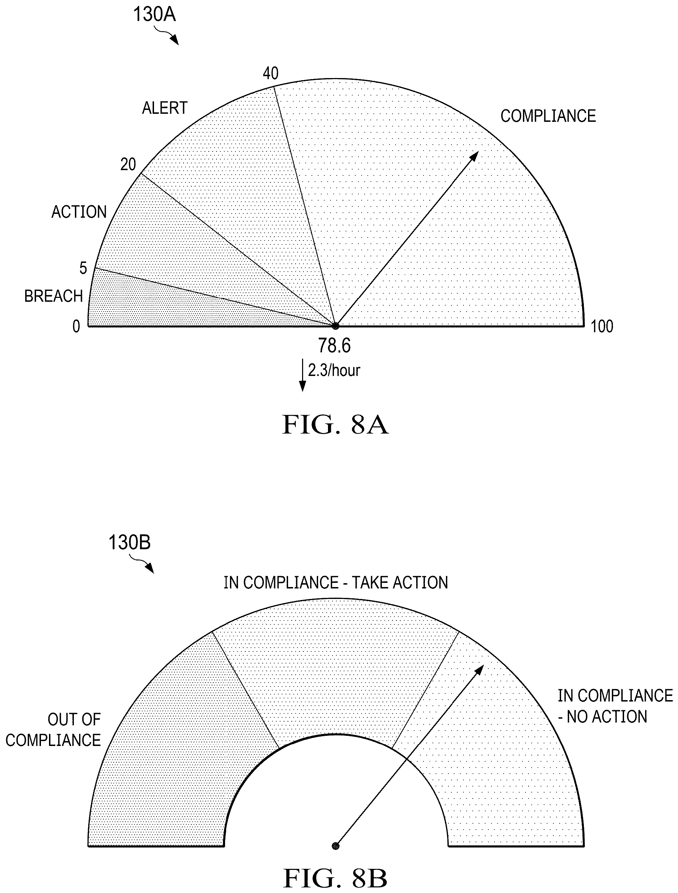

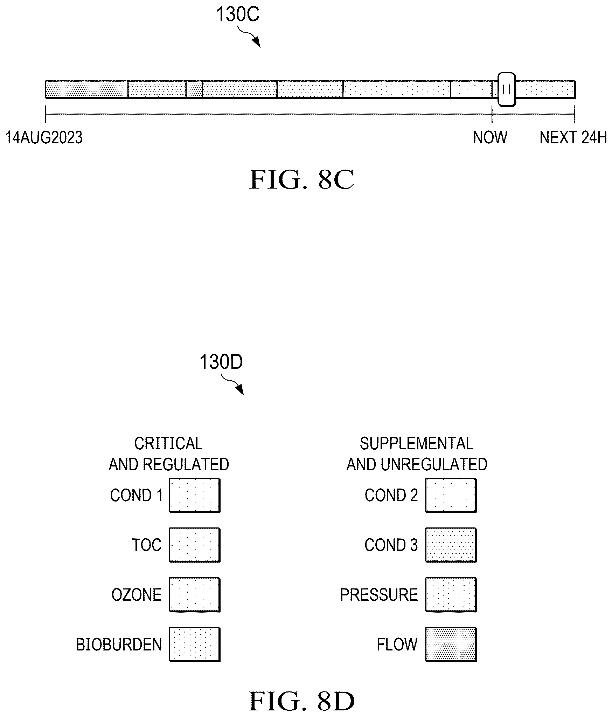

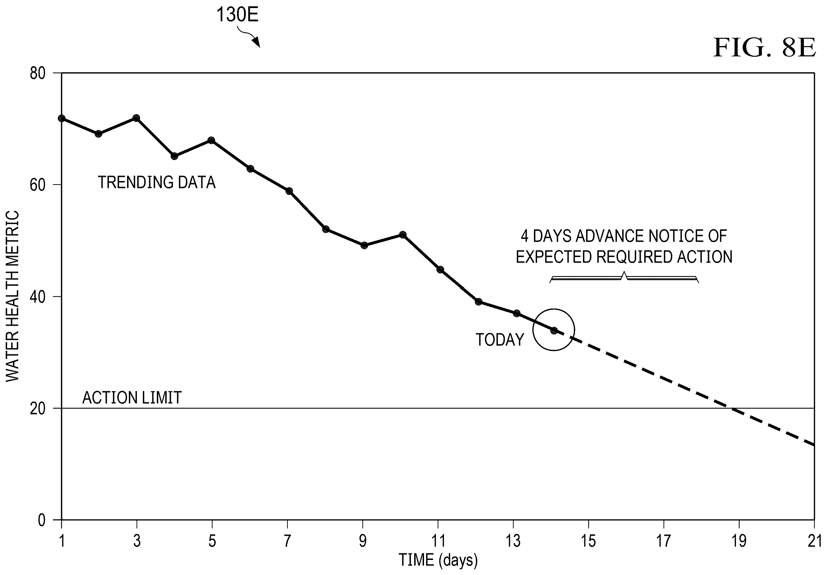

In exemplary embodiments, without limitation, a graphical representation 130 generated, at least in part by the analysis subsystem 110, may be as shown in one or more of **FIGURE 8A** through **FIGURE 8E** (130A, 130B, 130C, 130D, 130E, respectively). The output may comprise only a composite indication of the state of the water system 100 and/or water purification subsystem 108 (e.g., figures 8A, 8B), a characteristic specific state of the water system 100 and/or water purification subsystem 108 (e.g., figure 8D) for some or all characteristics/components thereof, a historical indication of the aforementioned (e.g., figures 8C, 8E; such as on a composite and/or characteristic/component specific basis), a future predicted state thereof (e.g., figure 8A, 8C, 8E), combinations thereof, or the like. In figure 8B, shading may be utilized to indicate movement of the arrow over time. The zones and/or status may be color coded and/or labeled. In figure 8C, a slider may demonstrate a current time and/or a user selected time on a time scale, by way of non-limiting example. This may include a future time, which may be forecasted by data extrapolation techniques, such as but not limited to, regression and/or extrapolation. In figure 8A, trended data and an accompanying directional arrow may be provided.

Data received over time, such as on a CWQM basis and/or on a characteristic specific basis, may be extrapolated to predict a future state of the water system or characteristic (e.g., figures 8A, 8C, 8E). Preemptive alerts may be made where a state of the water system and/or on a characteristic is expected to change relative to the associated threshold(s) over a predetermined period of time (e.g., one or more minutes, hours, days, weeks, etc.) and/or move zone(s). The triggers and/or types for such alerting may be predefined and/or user specified.

The alerts may be provided as electronic notifications and/or reports, by way of non-limiting example. These notifications and/or reports may comprise any one or more of the outputs shown and/or described herein. These notifications and/or reports may be transmitted to one or more remote electronic devices, such as smartphones, computers, servers, tablets, industrial controllers (e.g., PLCs/DCSs), combinations thereof, or the like. Data transmission and/or reports may be communicated by way of one or more conventional rely devices. Alternatively, or additionally, any or all of the outputs and/or reports shown and/or described herein may be accessed on an on-demand basis, such as through such one or more remote electronic devices.

The analysis subsystem 110 may be configured to accept simulated data, such as to determine simulated characteristics states and/or CWQM. This may be used to simulate various conditions that may be experienced, and may be particularly useful, without limitation, for judging sensitivity and/or establishing user modified thresholds.

Listing of certain embodiments - provided hereinafter is a listing of certain non-limiting examples of embodiments of the technology.

A1. A system for dynamically determining water quality for a water system, said system comprising:
a controller comprising, and/or having access to, one or more non-transitory electronic storage devices comprising thresholds, where one of said thresholds ("composite threshold") is specific to a composite water quality metric ("CWQM") and a plurality of said thresholds ("characteristic thresholds") are each specific to a respective one of characteristics of a sample of the water system, where each of said characteristic thresholds separate zones that indicate a state of the water system with respect to the associated characteristic, where the composite threshold separates zones that indicate a state of the water system with respect to the CWQM, and software instructions, which when executed, configure one or more processors to:
   receive data from sensors for measuring the characteristics, the data indicating measurements of the characteristics;
   determine, for each of the characteristics, a proximity of an associated one or ones of the measurements to an associated one of the characteristic thresholds;
   use the determined proximities to adjust the CWQM;
   determine a current state of the water system based on the adjusted CWQM relative to the composite threshold; and
   cause a representation of the current state of the water system to be communicated to one or more electronic devices.

A2. The system of embodiment A1 wherein: one or more of the characteristic thresholds are associated with one or more regulated water characteristics, in particular regulated water characteristics for purified water.

A3. The system of any one of embodiments A1-A2 wherein: the software instructions, when executed, further configure the one or more processors to: associate each of the one or ones of the measurements with a respective one of a plurality of subsets of characteristics.

A4. The system of embodiment A3 wherein: the plurality of subsets of characteristics comprises a first subset of characteristics associated with a regulated water characteristic and a second subset of characteristics associated with an unregulated water characteristic.

A5. The system of any one of embodiments A1-A4 wherein: the software instructions, when executed, further configure the one or more processors to: adjust a contribution of a characteristic to the CWQM in dependency on the determined proximity of the associated one or ones of the measurements to the associated one of the characteristic thresholds.

A6. The system of any one of embodiments A1-A5 wherein: the software instructions, when executed, further configure the one or more processors to: for each of the characteristics, determine the proximity for the characteristic in relation to the proximities determined for the other characteristics for determining the contribution of the characteristic to the composite water quality metric.

A7. The system of embodiment A6 wherein: the software instructions, when executed, further configure the one or more processors to: apply an expert system algorithm to determine the CWQM from the determined relations.

A8. The system of any one of embodiments A1-A7, wherein: the software instructions, when executed, configure the one or more processors to: for each of the characteristics, adjust a weighting assigned to the associated characteristic based on the proximity such that the weighting increases as the proximity decreases.

A9. The system of any one of embodiments A1-A8, in particular of embodiment A8, wherein: the software instructions, when executed, further configure the one or more processors to: where the one or ones of the measurements associated with any one or more of a first subset of characteristics falls on an adverse side of the associated threshold, apply all weighting to a most unfavorable one of the one or ones of the measurements associated with the first subset of characteristics.

A10. The system of any one of embodiments A8-A9, in particular of embodiment A8, wherein: the software instructions, when executed, further configure the one or more processors to: where the one or ones of the measurements associated with all of the first subset of characteristics falls on a favorable side of the associated threshold, apply all weighting to the one or ones of the measurements associated with all of the first subset of characteristics, or apply all weighting to the one or ones of the measurements, including the one or ones of the measurements associated with all of the first subset of characteristics and measurements associated with any one or more of a second subset of characteristics, based on the proximity of each of the measurements to the associated characteristic threshold.

A11. The system of embodiment A9 or A10 wherein:
each of the first subset of the characteristics is associated with a regulated water characteristic; and
where one or more of the measurements is associated with any one or more of a or, as the case may be, the second subset of characteristics, each of the second subset of the characteristics is associated with an unregulated water characteristic.

A12. The system of any one of embodiments A1-A11 wherein: the software instructions, when executed, further configure the one or more processors to: where the one or ones of the measurements associated with all of the first subset of characteristics falls on a favorable side of the associated threshold, or where the one or ones of the measurements associated with all of the first subset of characteristics falls on the favorable side of the associated threshold and the one or ones of the measurements associated with at least one of the second subset of characteristics falls on the adverse side of the associated threshold, scale the one or ones of the measurements associated with the at least one of the second subset of characteristics, or the one or ones of the measurements associated with all of the second subset of characteristics ("unfavorable supplemental parameters"), such that the unfavorable supplemental parameters is set to a zero value where the unfavorable supplemental parameters are at or on the unfavorable side of the associated threshold.

A13. The system of any one of embodiments A1-A12, in particular of A8 and A9-A12 when including the features of A8, wherein: the software instructions, when executed, further configure the one or more processors to: adjust the weightings on a zone-specific scale.

A14. The system of any one of embodiments A1-A13 wherein:
at least two of said characteristic thresholds are associated with each of said characteristics, thereby defining at least three zones for each of said characteristics; and
the zones comprise:
   an out-of-compliance zone, a take action zone, and a no action required zone; or
   the zones comprise a breach, action, alert, and compliance zone.

A15. The system of any one of embodiments A1-A14 wherein: the representation comprises a graphical representation of the zones, where the current state of the water system is graphically indicated as a location within one of the zones.

A16. The system of any one of embodiments A1-A15 wherein:
the data is received from the sensors on a continuous, at least substantially real-time basis;
the software instructions, when executed, configure the one or more processors to: analyze the current state of the water system on a continuous, at least substantially real-time basis as the data is received.

A17. The system of any one of embodiments A1-A16 wherein:
the software instructions, when executed, further configure the one or more processors to: store the current state of the water system on a time dependent basis such that current states of the water system are stored over time; and
the representation comprises a time-based historical representation of the current state of the water system.

A18. The system of any one of embodiments A1-A17 wherein: the software instructions, when executed, further configure the one or more processors to: determine, separately for each of the characteristics, respectively, a current state of the respective characteristic based on the proximity of the associated one or ones of the measurements to the associated one of the characteristic thresholds; and
the representation comprises, separately for each of the characteristics, respectively, a representation of the current state of the respective characteristic.

A19. The system of any one of embodiments A1-A18 wherein:
the water system comprises one or more valves and pumps;
the controller is in electronic communication with the one or more valves and pumps; and
the software instructions, when executed, further configure the one or more processors to: where the current state of the water system is determined to be on an adverse side of the composite threshold, operate the one or more valves or pumps.

A20. The system of any one of embodiments A1-A19 wherein: the software instructions, when executed, further configure the one or more processors to: where the current state of the water system is determined to be on an adverse side of the composite threshold, cause an alert to be generated at the one or more electronic devices.

A21. The system of any one of embodiments A1-A20 wherein: the software instructions, when executed, further configure the one or more processors to:
perform an extrapolation of the measurements for some or all of the characteristics;
determine a predicted future state of the water system based, at least in part, on a proximity of the extrapolated measurements to the characteristic thresholds associated to the some or all of the characteristics; and
provide, at the representation, an indication of the predicted future state of the water system.

A22. The system of any one of embodiments A1-A21 wherein:
at least some of the thresholds are specific to geographic regions; and
the software instructions, when executed, further configure the one or more processors to:
   receive, retrieve, or prompt an indication of a desired one of the geographic regions; and
   apply the thresholds specific to the desired one of the geographic regions.

A23. The system of any one of embodiments A1-A22 wherein:
one or more of the thresholds are user specified or modified; and
the software instructions, when executed, further configure the one or more processors to:
   receive, retrieve, or prompt user input establishing or updating the one or more of the thresholds from the one or more electronic devices; and
   apply the one or more of the thresholds as specified in the user input.

A24. The system of any one of embodiments A1-A23 wherein: the software instructions, when executed, further configure the one or more processors to:
receive simulated data indicating simulated measurements for some or all of the characteristics;
determine a simulated state of the water system based, at least in part, on a proximity of the simulated measurements to the thresholds associated to the some or all of the characteristics; and
provide, at the representation, an indication of the simulated state of the water system.

A25. The system of any one of embodiments A1-A24 wherein: the system comprises the sensors for measuring the characteristics and the controller is in electronic communication with each of the sensors.

A26. The system of embodiment A25 wherein:
the water system comprises a purified water manufacturing subsystem, a storage tank, a first feed line from the purified water source into the storage tank, a second feed line from the storage tank to at least one point of use output, and a return line from the at least one point of use output to the storage tank; and
the sensors are located, at least in part, along one or more of: the return line, the first feed line, and the second feed line.

B1. A computer-implemented method for dynamically determining water quality for a water system, said computer-implemented method comprising:
receiving, at a controller, data from sensors for measuring the characteristics, the data indicating measurements of the characteristics, wherein the controller comprises, and/or has access to, one or more non-transitory electronic storage devices comprising thresholds, where one of said thresholds ("composite threshold") is specific to a composite water quality metric ("CWQM") and a plurality of said thresholds ("characteristic thresholds") are each specific to a respective one of characteristics of a sample of the water system, where each of said characteristic thresholds separate zones that indicate a state of the water system with respect to the associated characteristic, where the composite threshold separates zones that indicate a state of the water system with respect to the CWQM;
determining, by way of the controller, for each of the characteristics, a proximity of an associated one or ones of the measurements to an associated one of the characteristic thresholds;
using, by way of the controller, the determined proximities to adjust the CWQM;
determining, by way of the controller, a current state of the water system based on the adjusted CWQM relative to the composite threshold; and
causing, by way of the controller a representation of the current state of the water system to be communicated to one or more electronic devices.

C1. A non-transitory electronic storage medium comprising software instruction for dynamically determining water quality for a water system and comprising, and/or having access to, thresholds, where one of said thresholds ("composite threshold") is specific to a composite water quality metric ("CWQM") and a plurality of said thresholds ("characteristic thresholds") are each specific to a respective one of characteristics of a sample of the water system, where each of said characteristic thresholds separate zones that indicate a state of the water system with respect to the associated characteristic, where the composite threshold separates zones that indicate a state of the water system with respect to the CWQM, wherein said software instructions, which when executed, configure one or more processors to:
receive data from sensors for measuring the characteristics, the data indicating measurements of the characteristics;
determine, for each of the characteristics, a proximity of an associated one or ones of the measurements to an associated one of the characteristic thresholds;
use the determined proximities to adjust the CWQM;
determine a current state of the water system based on the adjusted CWQM relative to the composite threshold; and
cause a representation of the current state of the water system to be communicated to one or more electronic devices.

Any embodiment of the present invention may include any of the features of the other embodiments of the present invention. The exemplary embodiments herein disclosed are not intended to be exhaustive or to unnecessarily limit the scope of the invention. The exemplary embodiments were chosen and described in order to explain the principles of the present invention so that others skilled in the art may practice the invention. Having shown and described exemplary embodiments of the present invention, those skilled in the art will realize that many variations and modifications may be made to the described invention. Many of those variations and modifications will provide the same result and fall within the spirit of the claimed invention.

Certain operations described herein may be performed by one or more electronic devices. Each electronic device may comprise one or more processors, electronic storage devices, executable software instructions, combinations thereof, and the like configured to perform the operations described herein. The electronic devices may be general purpose computers or specialized computing devices. The electronic devices may comprise personal computers, smartphones, tablets, databases, servers, or the like. The electronic connections and transmissions described herein may be accomplished by one or more wired or wireless connectivity components (e.g., routers, modems, ethernet cables, fiber optic cables, telephone cables, signal repeaters, and the like) and/or networks (e.g., internets, intranets, cellular networks, the world wide web, local area networks, and the like). The computerized hardware, software, components, systems, steps, methods, and/or processes described herein may serve to improve the speed of the computerized hardware, software, systems, steps, methods, and/or processes described herein. The electronic devices, including but not necessarily limited to the electronic storage devices, databases, controllers, or the like, may comprise and/or be configured to hold, solely non-transitory signals.

## Claims

1. A system (102) for dynamically determining water quality for a water system (10, 100), said system comprising:
a controller (111) comprising, and/or having access to, one or more non-transitory electronic storage devices (115) comprising thresholds, where one of said thresholds ("composite threshold") is specific to a composite water quality metric ("CWQM") and a plurality of said thresholds ("characteristic thresholds") are each specific to a respective one of characteristics of a sample of the water system, where each of said characteristic thresholds separate zones that indicate a state of the water system (10, 100) with respect to the associated characteristic, where the composite threshold separates zones that indicate a state of the water system (10, 100) with respect to the CWQM, and software instructions, which when executed, configure one or more processors (117) to:
receive data from sensors (114, 116, 118) for measuring the characteristics, the data indicating measurements of the characteristics;
determine, for each of the characteristics, a proximity of an associated one or ones of the measurements to an associated one of the characteristic thresholds;
use the determined proximities to adjust the CWQM;
determine a current state of the water system (10, 100) based on the adjusted CWQM relative to the composite threshold; and
cause a representation (130A-130E) of the current state of the water system (10, 100) to be communicated to one or more electronic devices (119).

2. The system (102) of claim 1 wherein:
one or more of the characteristic thresholds are associated with one or more regulated water characteristics, in particular regulated water characteristics for purified water.

3. The system (102) of claim 1 or 2 wherein:
the software instructions, when executed, further configure the one or more processors (117) to:
adjust a contribution of a characteristic to the CWQM in dependency on the determined proximity of the associated one or ones of the measurements to the associated one of the characteristic thresholds; and
for each of the characteristics, determine the proximity for the characteristic in relation to the proximities determined for the other characteristics for determining the contribution of the characteristic to the composite water quality metric.

4. The system (102) of claim 3 wherein:
the software instructions, when executed, further configure the one or more processors (117) to: apply an expert system algorithm to determine the CWQM from the determined relations.

5. The system (102) of any one of claims 1-4, wherein:
the software instructions, when executed, configure the one or more processors (117) to: for each of the characteristics, adjust a weighting assigned to the associated characteristic based on the proximity such that the weighting increases as the proximity decreases.

6. The system (102) of claim 5, wherein:
the software instructions, when executed, further configure the one or more processors (117) to: where the one or ones of the measurements associated with any one or more of a first subset of characteristics falls on an adverse side of the associated threshold, apply all weighting to a most unfavorable one of the one or ones of the measurements associated with the first subset of characteristics.

7. The system (102) of claim 5 wherein:
the software instructions, when executed, further configure the one or more processors (117) to: where the one or ones of the measurements associated with all of a first subset of characteristics falls on a favorable side of the associated threshold, apply all weighting to the one or ones of the measurements associated with all of the first subset of characteristics, or apply all weighting to the one or ones of the measurements, including the one or ones of the measurements associated with all of the first subset of characteristics and measurements associated with any one or more of a second subset of characteristics, based on the proximity of each of the measurements to the associated characteristic threshold.

8. The system (102) of any one of claims 4-7 wherein:
the software instructions, when executed, further configure the one or more processors (117) to: adjust the weightings on a zone-specific scale.

9. The system (102) of any one of claims 1-8 wherein:
at least two of said characteristic thresholds are associated with each of said characteristics, thereby defining at least three zones for each of said characteristics; and
the zones comprise:
an out-of-compliance zone, a take action zone, and a no action required zone; or
the zones comprise a breach, action, alert, and compliance zone.

10. The system (102) of any one of claims 1-9 wherein:
the water system (10, 100) comprises one or more valves and pumps;
the controller (111) is in electronic communication with the one or more valves and pumps; and
the software instructions, when executed, further configure the one or more processors (117) to: where the current state of the water system (10, 100) is determined to be on an adverse side of the composite threshold, operate the one or more valves or pumps.

11. The system (102) of any one of claims 1-10 wherein:
the software instructions, when executed, further configure the one or more processors (117) to:
perform an extrapolation of the measurements for some or all of the characteristics;
determine a predicted future state of the water system (10, 100) based, at least in part, on a proximity of the extrapolated measurements to the characteristic thresholds associated to the some or all of the characteristics; and
provide, at the representation (130A, 130C, 130E), an indication of the predicted future state of the water system.

12. The system (102) of any one of claims 1-11 wherein:
the software instructions, when executed, further configure the one or more processors (117) to:
receive simulated data indicating simulated measurements for some or all of the characteristics;
determine a simulated state of the water system (10, 100) based, at least in part, on a proximity of the simulated measurements to the thresholds associated to the some or all of the characteristics; and
provide, at the representation, an indication of the simulated state of the water system.

13. The system (102) of any one of claims 1-12 wherein:
the system comprises the sensors (114, 116, 118) for measuring the characteristics and the controller (111) is in electronic communication with each of the sensors.

14. A computer-implemented method for dynamically determining water quality for a water system (10, 100), said computer-implemented method comprising:
receiving, at a controller (111), data from sensors (114, 116, 118) for measuring characteristics, the data indicating measurements of the characteristics, wherein the controller (111) comprises, and/or has access to, one or more non-transitory electronic storage devices (115) comprising thresholds, where one of said thresholds ("composite threshold") is specific to a composite water quality metric ("CWQM") and a plurality of said thresholds ("characteristic thresholds") are each specific to a respective one of characteristics of a sample of the water system, where each of said characteristic thresholds separate zones that indicate a state of the water system with respect to the associated characteristic, where the composite threshold separates zones that indicate a state of the water system with respect to the CWQM;
determining, by way of the controller (111), for each of the characteristics, a proximity of an associated one or ones of the measurements to an associated one of the characteristic thresholds;
using, by way of the controller (111), the determined proximities to adjust the CWQM;
determining, by way of the controller (111), a current state of the water system based on the adjusted CWQM relative to the composite threshold; and
causing, by way of the controller (111), a representation (130A-130E) of the current state of the water system to be communicated to one or more electronic devices (119).

15. A non-transitory electronic storage medium comprising software instructions for dynamically determining water quality for a water system (10, 100) and comprising, and/or having access to, thresholds, where one of said thresholds ("composite threshold") is specific to a composite water quality metric ("CWQM") and a plurality of said thresholds ("characteristic thresholds") are each specific to a respective one of characteristics of a sample of the water system, where each of said characteristic thresholds separate zones that indicate a state of the water system with respect to the associated characteristic, where the composite threshold separates zones that indicate a state of the water system with respect to the CWQM, wherein said software instructions, which when executed, configure one or more processors (117) to:
receive data from sensors (114, 116, 118) for measuring the characteristics, the data indicating measurements of the characteristics;
determine, for each of the characteristics, a proximity of an associated one or ones of the measurements to an associated one of the characteristic thresholds;
use the determined proximities to adjust the CWQM;
determine a current state of the water system based on the adjusted CWQM relative to the composite threshold; and
cause a representation (130A-130E) of the current state of the water system to be communicated to one or more electronic devices (119).
